Europäisches Patentamt

European Patent Office

Office européen des brevets

(11) Publication number: **0 332 147 B1**

## EUROPEAN PATENT SPECIFICATION

(45) Date of publication of patent specification: **16.06.93** (51) Int. Cl.5: **A61K 47/02, A61K 47/14**

(21) Application number: **89104017.2**

(22) Date of filing: **07.03.89**

(54) Compositions with enhanced penetration.

(30) Priority: **08.03.88 US 165322**

(43) Date of publication of application:
**13.09.89 Bulletin 89/37**

(45) Publication of the grant of the patent:
**16.06.93 Bulletin 93/24**

(84) Designated Contracting States:
**AT BE CH DE ES FR GB GR IT LI LU NL SE**

(56) References cited:
**EP-A- 0 137 978**
**GB-A- 2 107 588**

(73) Proprietor: **WARNER-LAMBERT COMPANY**
**201 Tabor Road**
**Morris Plains New Jersey 07950(US)**

(72) Inventor: **Mahjour, Majid**
**82 Kochlas Drive**
**Netcong New Jersey 07857(US)**
Inventor: **Mauser, Bernadette**
**462 Kingland Avenue**
**Lyndhurst New Jersey 07071(US)**
Inventor: **Rashidbaigi, Zahra A.**
**33 Ferncliff Road**
**Morris PLains New Jersey 07950(US)**

(74) Representative: **Mansmann, Ivo**
**c/o Gödecke AG - Patentabteilung Postfach**
**569 Mooswaldallee 1-9**
**W-7800 Freiburg (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.6/3.3.1)

**Description**

Diphenhydramine, hydrochloride (Benadryl®, or 2-(diphenylmethoxy) N, N-dimethylethylamine), is known to be useful for its antihistaminic, anticholinergic, antitussive, antiemetic, and sedative properties. The compound and its preparation are described in US Patent 2,241,714.

The base is a liquid and its salts have acceptable solubilities in standard liquid media. Thus, diphenhydramine-based drugs are conventionally used in dosage forms such as oral and parenteral. However, there are undesirable digestive side effects possible with oral formulations. Generally there are compliance problems with parenterals.

Diphenhydramine is a nonprescription drug widely used alone or in combination with other drugs as an effective antihistamine with a sedative side effect. Carruthers, et al (Clin Pharmacol Ther 1978; 23:375-382) showed that the sedative side effect of diphenhydramine hydrochloride could be eliminated if the blood concentration remains in the range of 25 to 50 ng/ml (equivalent to 21.9 to 43.9 ng/ml diphenhydramine base). This indicates that a sustained dosage form which could provide such constant blood levels would be a very useful and viable dosage form in the treatment of allergy.

Tetrahydroaminoacridine, 1,2,3,4-tetrahydro-9-acridinamine Tacrine, THA), a very old compound is known to be useful as a respiratory stimulant and has anticholinesterase activity. It has been shown that THA improves the amnesia characteristic of Alzheimer's disease (Brinkman and Gershon, 1983; Flood et al, 1985; Rathman and Conner, 1984; McGeer, 1984).

Tazifylline is known to be useful as an antihistamine. It is covered in US Patent 4,374,835.

Atenolol, 4-[2-hydroxy-3-[(1-methylethyl)amino]-propoxy]benzeneacetamide, is covered in US Patents 3,663,607 and 3,836,671. It is a $\beta$-adrenergic blocker.

2-Methoxy-4-[2-(5-methyl-1H-pyrazol-3-yl)ethenyl]-phenol (PD 123,124) has antiasthma, antiallergy, antiinflammtory, antipsoriatic, analgesic, and cardiovascular activities.

While the above drugs are highly efficacious, their use is subject to such problems as dose dumping and high drug use requirements.

United States Patent No. 4,611,008 discloses the use of neutral oil like Miglyol®-812 or Miglyol®-829 in a coronary-active gel-containing preparation. Miglyol® is a mixture of triglycerides containing saturated plant fatty acids of a chain length of $C_8$ to $C_{12}$ (Angewandte Biopharmazie, Wiss. Verl.Ges. Stuttgart 1973, p. 314).

United States Patent No. 4,331,651 covers in part, caprylic/capric acid -1,2-propanediol diester used as a release-promoting substance used in a silicone rubber carrier for an active ingredient.

United States Patent No. 4,336,243 covers a microsealed transdermal delivery pad for nitroglycerin administration which contains a silicon matrix having microsealed compartments of silicone rubber mixed with a hydrophilic solvent system, the solvent system can contain a saturated coconut oil such as miglyol® oil which improves the transport and absorption of the nitroglycerin.

It has been discovered that the therapeutically active agents diphenhydramine, tetrahydroaminoacridine, atenolol, tazifylline and 2-methoxy-4-[2-(5-methyl-1H-pyrazol-3-yl)ethenyl]phenol in combination with a solvent Miglyol® oil, especially Miglyol® 840, which is a propylene glycol diester of saturated vegetable fatty acids of chain length $C_8$ to $C_{10}$ and if desired a gelling agent such as silicic acid (Aerosol® 200) have a higher flux value than formulations without Miglyol® oil when applied on skin. Aerosil® consists of amorph spheric particles of silicic acid prepared by hydrolysis of Silicium tetrachloride.

EP-A 0 137 978 covers the preparation of gel-compositions of coronary active nitroesters. In order to avoid the separation of oily components it has been suggested to exchange a part of the hydrophilic $SiO_2$ adjuvant by hydrophobic $SiO_2$. This document, however, does not teach the unexpected transmembranal usefulness of the specific compositions proposed by the present invention.

Gastrointestinal problems associated with some drugs which are administered orally are thus eliminated. The gradual release of a drug via a membranal tissue minimizes the risk of dose dumping and other side effects.

In addition, the use of the present invention would result in a reduction in overall drug load dose. Furthermore, a patch or other transmembranal device serves as a reminder to the patient to administer the proper dosage.

These and other advantages of the invention will become apparent upon consideration of the following description of the invention.

The invention is a pharmaceutical composition for use in transmembranal administration of drugs comprising:

(a) 0.2% to 5% by weight of therapeutically active medicament selected from diphenhydramine, tetrahydroaminoacridine, atenolol, tasifylline, 2-methoxy-4-[2-(5-methyl-1H-pyrazol-3-yl)ethenyl] phenol or a pharmaceutically acceptable salt thereof,

(b) up to 99.8% by weight of a propylene glycol diester of caprylic and capric acids.

(c) up to 15% by weight silicic acid.

A further subject of the invention is a process for manufacturing a pharmaceutical composition for use in transmembranal administration of drugs characterized in that 0.2% to 5% by weight of a medicament selected from diphenhydramine, tetrahydroaminoacridine, atenolol, tazifylline, 2-methoxy-4-[2-(5-methyl-1H-pyrazol-3-yl)ethenyl] phenol or a pharmaceutically acceptable salt thereof is combined with up to 99.8 weight% of a propylene glycol diester of caprylic and capric acids and up to 15% by weight silicic acid.

In a preferred embodiment of the instant invention, a transmembranal composition of diphenhydramine or a pharmaceutically acceptable salt thereof is combined with Miglyol®, a gelling agent and an alcohol in the form of a gel. The gel would be on a controlling or a noncontrolling membrane compatible with manufacturing processes and capable of providing the desired flux of the active ingredient. Microporous membranes, silicone-membranes (Silastic®), and/or polyurethane (with polyester or polyether backbone) membranes among others may be used.

The side effects often associated with the use of diphenhydramine in oral and parenteral formulations can be overcome by administration of the drug via topical application to body membranes for absorption into the system.

Transdermal delivery systems are not always efficacious due to such factors as the failure of the drug to penetrate sufficiently the cutaneous membrane and enter the body to produce therapeutic systemic effects. The present invention uses a novel penetration enhancer to deliver efficacious amounts of the desired drug to the body. The compositions and methods of the instant invention have several advantages over conventional systems for the delivery of drugs. One principal advantage concerns the undesirable side effects associated with administration via dosage forms which are swallowed or injected. For instance, the nausea and/or other gastrointestinal discomfort associated with liquids and solids which are swallowed is eliminated when transmembrane administration is employed. The compositions of the instant invention are introduced into the body via various membranes, for example, transdermally, buccally, rectally, and nasally.

Also, the storage and transportation problems associated with liquid dosage forms are generally eliminated when transmembrane administration is employed. These are usually creams, gels, and solid suppositories. In the compositions of the instant invention the topical delivery of the drugs has several advantages. One such is the gradual release of the drug via membranal tissue, for example, on the skin or in the nasal passages, minimizes the risk of dose dumping and can also reduce the overall drug loading dose. Also, a patch or other transdermal device serves as a reminder to the patient to administer the proper dosage of the drug.

These and other advantages of the invention will be apparent upon the following description of the invention.

All percentages recited herein are weight percentages based on total composition weight unless otherwise specified.

The term diphenhydramine and pharmaceutically acceptable salts thereof is intended to include all forms of diphenhydramine and/or its analogs which have medical utility. Thus, diphenhydramine and its hydrochloride salt are contemplated. Mixtures may also be used.

While the use of a diphenhydramine-based drug is an example of a transmembranal formulation, the use of other beneficial substances as mentioned above is also contemplated.

Generally the drug component will comprise 0,2 to 5% by weight of the total composition.

Other components such as alcohol and a gelling agent are also contemplated. A preffered gelling agent is Aerosil® 200.

Aerosil® 200 is a very pure silicic acid with a content of >99.8% $SiO_2$, $Al_2O_3$>0.05, $TiO_2$>0.03, HCl>0.01 and a moisture of about 1.5%. The pH-value of a 4% water dispersion is 3.9 ± 0.3. The BET-surface is 200 ±25 and the medium size of the primary particles is 16 $\mu$m (Fiedler, Lexikon der Hilfsstoffe für Pharmazie, Kosmetik und angrenzende Gebiete, Editio Cantor KG, Aulendorf i. Wuertt., 1971, page 20).

All acceptable excipients, antioxidants, preservatives, including fatty acid esters as for example hexadecanoic methyl ester of Wickenol® 535 and vitamin E may be included in the composition of the invention.

A controlling membrane is also contemplated. Preferred membranes include microporous membranes, silicone membranes (Silastic® ) or polyurethane membranes.

The above composition is administered by contracting the drug with a vehicle to produce a transmembranal formulation and applying that formulation in a suitable device to body membrane for absorption therethrough.

Other conventional adjuncts, for example, colorants, perfumes, stabilizers, and the like can also be employed in compositions of the present invention.

The permeation-enhancing portion of the instant invention is a substance which functions to assist in the migration of the drug component(s) through the membranes and into the bloodstream. Thus, any agent(s) which function to hasten and/or to regulate the transmembranal passage or systemic release of drug(s) can be used in combination with a propylene glycol diester of caprylic and capric acids.

Generally the solvent, the permeation-enhancing component, will comprise about 0.1 to 99.9%, preferably to 80%, and most preferably 50 to 80% by weight of the total composition. The action ingredient permeates through the skin as a neat drug (base) or in formulations containing a solvent.

The solvent component will comprise at least one solvent for the drug component. Useful solvents include but are not limited to Miglyol® oils, alcohol, IPM, and the like. Mixtures of two or more are also usable. Preferably the solvent is Miglyol® 840. The solvent component will comprise 0.1 to 99.8%, preferably from 50 to 80% and most preferably from 50 to 70% by weight of the total composition.

The term solvent is intended to include that portion of the formulation which provides the flux of the active ingredient.

The following example illustrates one embodiment of the invention. It is not intended to limit the scope of the invention in any way:

The drug-containing gel composition:

| Ingredients | Percent (%) w/w |
|---|---|
| Diphenhydramine Base | 20.0 |
| Miglyol® 840 | 73.0 |
| Wickenol® 535 | 0.5 |
| Vit. E Alcohol USP | 0.05 |
| Aerosil® 200 | 6.45 |

Both Silastic® membrane and polyurethane membranes (with polyester or polyether backbone) were evaluated. In addition the effect of the gelling agent Aerosil® 200 on diphenhydramine flux was determined.

Pharmaceutical compositions containing Miglyol® neutral oils are useful in effecting transdermal delivery of a therapeutic dose of an active drug to the system of mammals. Tables 1-5 below show a series of comparative in vitro diffusion studies illustrating the usefulness of Miglyol® oils, e.g., Miglyol® 840 (Dynamit Nobel Chemicals) as effectively enhancing the penetration of different drugs across biological membranes. Hairless mouse skin was used.

Miglyol® neutral oils, e.g., Miglyol® 812, 810, 818, and 840) are esters of medium chain fatty acids also called fractionated cocoanut oil.

Miglyol® neutral oils are completely stable against oxidation, tasteless and odorless, soluble in alcohol, nonirritating to the skin.

4

## TABLE I

### Comparison of Miglyol® 840's Effect on the Permeation of Tazifylline with Other Skin Permeation Enhancers Across Hairless Mouse Skin

| Formulation | Donor Conc.* (mg/ml) | Flux† ($\mu g/cm^2/h$) | Lag Time (h) | Permeability‡ (cm/sec)x$10^7$ |
|---|---|---|---|---|
| LA:PG 10:90 | 6.54 | 20.48 | 1.2 | 8.70 |
| LA:Miglyol® 840 10:90 | 14.37 | 33.68 | 8.47 | 6.51 |
| DMSO:$H_2O$ 80:20 | 5.96 | 25.08 | 15.97 | 11.69 |
| ETOH:Miglyol® 840 20:80 | 52.13 | 290.58 | 0.77 | 15.48 |
| LA:PG:TA 20:30:50 | 46.07 | 104.66 | 8.01 | 6.31 |
| Miglyol®-Gel: Miglyol® 840:ETOH 66.6:26.7:6.7 | 15.08 | 37.33 | 3.23 | 6.88 |
| DPH:PG 50:50 | 16.3 | 7.81 | 10.45 | 1.33 |

\* saturated solution  † Average flux value  ‡ Estimated by dividing flux value by initial drug concentration

LA = Linoleic Acid
PG = Propylene Glycol
DMSO = Dimethyl Sulfoxide
TA = Triacetin
ETOH = Ethanol
DPH = Diphenhydramine

EP 0 332 147 B1

TABLE II

Effect of Miglyol® 840 on Diphenhydramine (DPH) Flux Value
Across Hairless Mouse Skin

| Formulation w/w | Flux† ($\mu g/cm^2/h$) | Lag Time (h) |
|---|---|---|
| 60:40 Miglyol® 840:DPH | 132 ± 21 | 0 |
| 70:30 Miglyol® 840:DPH | 118 ± 9 | 0 |
| 90:10 Miglyol® 840:DPH | 103 ± 18 | 0.2 ± 0.2 |
| 95:5 Miglyol® 840:DPH | 70 ± 19 | 0.3 ± 0.2 |
| Neat DPH | 94 | 0 |

† Average flux value

## TABLE III

### Effect of Miglyol® 840 on Enhancing the Penetration of PD 123,124 Across Hairless Mouse Skin

| Solvent System | Drug Conc. (mg/ml) | Flux Value† ($\mu g/cm^2/h$) | Lag Time (h) |
|---|---|---|---|
| Acetone | 5 | 6.46 ± 2.43 | 1.68 ± 0.67 |
| LA:PG:TA 20:30:50 w/w | 20 | 7.1 ± 5.8 | 2.65 ± 0.16 |
| Miglyol® 840:ETOH 80:20 w/w | 10 | 41.33 ± 7.68 | 2.2 ± 0.1 |

† Average flux value

EP 0 332 147 B1

## TABLE IV

### Skin Penetration Enhancement Effect of Miglyol® 840 on THA as Compared with Other Penetration Enhancers

| Solvent System | Percent (w/w) | Flux Value† ($\mu g/cm^2/h$) | Lag Time (h) |
|---|---|---|---|
| LA:PG:TA 20:30:50 w/w | 2 | 168.6 ± 1.9 | 2.9 ± 1.3 |
| LA:ETOH:$H_2O$ 5:70:25 w/w | 4 | 645.6 ± 87 | 6.5 ± 0.5 |
| DPH:PG 50:50 w/w | 4 | 102.45 ± 5.5 | 5.1 ± 0.56 |
| Miglyol® 840:ETOH 80:20 | 4 | 2011.5 ± 117 | 1.3 ± 0.2 |
| PG | 4 | 106.44 | 16.6 |

† Average flux value

EP 0 332 147 B1

EP 0 332 147 B1

## TABLE V

### Effect of Miglyol® 840 on Penetration of Atenolol
### Across Hairless Mouse Skin

| Solvent System | Percent (w/w) | Flux Value† ($\mu g/cm^2/h$) | Lag Time (h) |
|---|---|---|---|
| PG | 4 | 38.39 | 22.4 |
| DPH:PG 50:50 w/w | 4 | 148.7 ± 8.76 | 9.1 ± 2.2 |
| Miglyol® 840:ETOH 80:20 w/w | 1 | 358.01 ± 29 | 0.5 ± 0.16 |

† Average flux value

**Claims**
**Claims for the following Contracting States : AT, BE, CH, DE, FR, GB, IT, LI, LU, NL, SE**

1. A pharmaceutical composition for use in transmembranal administration of drugs comprising:
   (a) 0.2% to 5% by weight of therapeutically active medicament selected from diphenhydramine, tetrahydroaminoacridine, atenolol, tazifylline, 2-methoxy-4-[2-(5-methyl-1H-pyrazol-3-yl)ethenyl] phenol or a pharmaceutically acceptable salt thereof,
   (b) up to 99.8% by weight of a propylene glycol diester of caprylic and capric acids.
   (c) up to 15% by weight silicic acid.

2. A process for manufacturing a phaumaceutical composition for use in transmembranal administration of drugs characterized in that 0.2% to 5% by weight of a medicament selected from diphenhydramine, tetrahydroaminoacridine, atenolol, tazifylline, 2-methoxy-4-[2-(5-methyl-1H-pyrazol-3-yl)ethenyl] phenol or a pharmaceutically acceptable salt thereof is combined with up to 99.8 weight% of a propylene glycol diester of caprylic and capric acids and up to 15% by weight silicic acid.

**Claim for the following Contracting States : ES, GR**

1. A process for manufacturing a pharmaceutical composition for use in transmembranal administration of drugs characterized in that 0.2% to 5% by weight of a medicament selected from diphenhydramine, tetrahydroaminoacridine, atenolol, tazifylline, 2-methoxy-4-[2-(5-methyl-1H-pyrazol-3-yl)ethenyl] phenol or a pharmaceutically acceptable salt thereof is combined with up to 99.8 weight% of a propylene glycol diester of caprylic and capric acids and with up to 15% by weight of silicic acid.

**Patentansprüche**
**Patentansprüche für folgende Vertragsstaaten : AT, BE, CH, DE, FR, GB, IT, LI, NL, SE**

1. Pharmazeutische Zusammensetzung zur Verwendung für die transmembranale Verabreichung von Medikamenten, enthaltend:
   (a) 0,2 bis 5 Gew.-% eines therapeutisch wirksamen Medikamentes, ausgewählt aus Diphenhydramin, Tetrahydroaminoacridin, Atenolol, Tazifyllin, 2-Methoxy-4-[2-(5-methyl-1H-pyrazol-3-yl)ethenyl]-phenol, oder eines pharmazeutisch annehmbaren Salzes dieser Verbindungen;
   (b) bis zu 99,8 Gew.-% eines Propylenglycol-Diesters von Caprylsäure und Caprinsäure; und
   (c) bis 15 Gew.-% Kieselsäure.

2. Verfahren zur Herstellung einer pharmazeutischen Zusammensetzung zur Verwendung für die transmembranale Verabreichung von Medikamenten, dadurch gekennzeichnet, dass man 0,2 bis 5 Gew.-% eines therapeutisch wirksamen Medikamentes, ausgewählt aus Diphenhydramin, Tetrahydroaminoacridin, Atenolol, Tazifyllin, 2-Methoxy-4-[2-(5-methyl-1H-pyrazol-3-yl)ethenyl]-phenol, oder eines pharmazeutisch annehmbaren Salzes dieser Verbindungen mit bis zu 99,8 Gew.-% eines Propylenglycol-Diesters von Caprylsäure und Caprinsäure und mit bis zu 15 Gew.-% Kieselsäure vereinigt.

**Patentanspruch für folgende Vertragsstaaten : ES, GR**

1. Verfahren zur Herstellung einer pharmazeutischen Zusammensetzung zur Verwendung für die transmembranale Verabreichung von Medikamenten, dadurch gekennzeichnet, dass man 0,2 bis 5 Gew.-% eines therapeutisch wirksamen Medikamentes, ausgewählt aus Diphenhydramin, Tetrahydroaminoacridin, Atenolol, Tazifyllin, 2-Methoxy-4-[2-(5-methyl-1H-pyrazol-3-yl)ethenyl]-phenol, oder eines pharmazeutisch annehmbaren Salzes dieser Verbindungen mit bis zu 99,8 Gew.-% eines PropylenglycolDiesters von Caprylsäure und Caprinsäure und mit bis zu 15 Gew.-% Kieselsäure vereinigt.

**Revendications**
**Revendication pour les Etats contractants suivants : AT, BE, CH, DE, FR, GB, IT, LI, LU, NL, SE**

1. Composition pharmaceutique pour l'administration transmembranaire de médicaments comprenant:
   (a) 0,2 à 5% en poids d'un médicament thérapeutiquement actif choisi parmi: diphenhydramine, tétrahydroaminoacridine, aténolol, tazifylline, 2-méthoxy-4-[2-(5-méthyl-1H-pyrazol-3-yl)éthényl]-phénol ou un de leurs sels pharmaceutiquement acceptables;

(b) jusqu'à 99,8% en poids d'un diester propylèneglycolique des acides caprylique et caprique;

(c) jusqu'à 15% en poids d'acide silicique.

2. Un procédé de fabrication d'une composition pharmaceutique pour l'administration transmembranaire de médicaments, caractérisé en ce que de 0,2 à 5% en poids d'un médicament, choisi parmi: diphenhydramine, tétrahydroaminoacridine, aténolol, tazifylline, 2-méthoxy-4-[2-(5-méthyl-1H-pyrazol-3-yl)éthényl]phénol ou un de leurs sels pharmaceutiquement acceptables, est combiné avec jusqu'à 99,8% en poids d'un diester propylèneglycolique des acides caprylique et caprique et jusqu'à 15% en poids d'acide silicique.

**Revendication pour les Etats contractants suivants : ES, GR**

1. Un procédé de fabrication d'une composition pharmaceutique pour administration transmembranaire de médicaments, caractérisé en ce que de 0,2 à 5% en poids d'un médicament, choisi parmi: diphenhydramine, tétrahydroaminoacridine, aténolol, tazifylline, 2-méthoxy-4-[2-(5-méthyl-1H-pyrazol-3-yl)-éthényl]phénol ou un de leurs sels pharmaceutiquement acceptables, est combiné avec jusqu'à 99,8% en poids d'un diester propylèneglycolique des acides caprylique et caprique et jusqu'à 15% en poids d'acide silicique.